# EUROPÄISCHE PATENTANMELDUNG

(11) **EP 2 390 309 A2**
(43) Veröffentlichungstag der Anmeldung: **30.11.2011**
(21) Anmeldenummer: 11003593.8
(22) Anmeldetag: 03.05.2011
(51) Int. Cl.: C12N 1/38, C12P 3/00, C10J 3/00

(54) **Verfahren zur Synthesegaserzeugung aus Biomasse**

(30) Priorität: 05.05.2010 DE 102010019351; 23.11.2010 DE 102010052060
(71) Anmelder: Rogmans, Maria, 47546 Kalkar (DE)
(72) Erfinder: Rogmans, Maria, 47546 Kalkar (DE)
(74) Vertreter: Schmidt, Karl Michael

(57) **Zusammenfassung**

Die Erfindung betrifft ein Verfahren, sowie eine Einrichtung zur Synthesegaserzeugung aus Biomasse, gemäß Oberbegriff der Ansprüche 1. Um hierbei durch Vorkonditionierung der Edukte (auch Biomasse) bessere Gaserträge oder Gasqualitäten zu erzielen, ist erfindungsgemäß vorgeschlagen, dass die Pflanzen, die die Biomasse darstellen, aquatische Pflanzen sind, bei welchen während des Wuchses der aquatischen Pflanzen über die definierte Zugabe des Elementes Bor oder Bor-Verbindungen zur Nährlösung der Stärkegehalt maximiert wird.

## Beschreibung

Die Erfindung betrifft ein Verfahren, sowie eine Einrichtung zur Synthesegaserzeugung aus Biomasse, gemäß Oberbegriff der Ansprüche 1.

Synthesegas ist bekanntermaßen ein kohlenmonoxid- und wasserstoffhaltiges Gas. Es wird entweder durch spezielle mikrobielle Fermentation aus Biomasse gewonnen, oder durch die klassische bekannte Kohle- oder Holzvergasung.

Der vorliegenden Erfindung liegt die Aufgabe zugrunde, für beide mögliche Verfahren durch bessere Vorkonditionierung der Edukte bessere Gaserträge oder Gasqualitäten zu erzielen.

Dies gestellte Aufgabe durch Anspruch 1 erfindungsgemäß gelöst.

Der Kern der Erfindung besteht hierbei darin, die Synthesegasqualität durch die definierte Zugabe des Elementes Bor oder Bor-Verbindungen beeinflussen zu können.
Bor-Verbindungen können beispielsweise Borate sein, wie bspw Silicoborate.

Dies ist in Verbindung mit den verwendeten SPEZIELLEN Pflanzen, nämlich aquatischen Pflanzen von erheblichem technologischen Vorteil. Der Grund dafür ist, dass die genannten Pflanzenarten, insbesondere Lemnacea und Spirodela, sowie alle bekannten Wasserlinsen alle 1 bis 5 Tage eine Massenverdopplung vornehmen.

**Damit ist die biochemische Reaktionszeit und damit dass Ergebnis einer Beigabe von Bor oder Bor-Verbindungen an der Pflanze so schnell vorliegend, wie bei keiner anderen Biomasse.**

Bei anderer Biomasse ist die Wuchszeit mindestens ein halbes Jahr. Bei bekannten holzigen Landpflanzen zur Energieerzeugung brauch es somit ein halbes Jahr, bis sich eine Düngemittelzugabe an der Pflanze umsetzt. Erst bei der Verwendung aquatischer Pflanzen sind die Wuchszeiten und Masseverdopplungszeiten bei nur einem Tag. Bei einer Düngemittelveränderung, d.h. hier der Bor-Gehalte, liegt die darauf inhaltstofflich veränderte Pflanze, hier Wasserlinse, innerhalb **eines Tages** vor. D.h. dass die so gesteuerte Biomasse innerhalb eines Folgetages vorliegt. Erst bei Einsatz solcher Pflanzen gewinnt dieser Effekt also an technologisch erheblicher Bedeutung.

Hierbei wird die Bor-Zugabe in Bezug auf zwei Verfahrensalternativen unterschiedlich dosiert.
1. Verfahrensalternative:
   Erzeugung von Synthesegas aus Biomasse (insbesondere aquatische Pflanzen) durch Heißverkokung der Biomasse zu Reinkohlenstoff und anschließendem Synthesegasprozess bekannter Art, wie bei der Kohlevergasung.
2. Verfahrensalternative:
   Erzeugung von Synthesegas durch sogenannte Synthesegas-Fermentation, durch bakterielle Umsetzung.

Bei der ersten Verfahrensalternative hat sich gezeigt, dass es besser ist, die aquatische Biomasse dahin vorzukonditionieren, dass diese mehr Stärke enthält.

Bei der zweiten Verfahrensalternative ist es besser, dass die aquatische Biomasse mehr Proteine enthält.

Im erstgenannten Fall werden daher die aquatischen Pflanzen mit einer Zugabe von nur 0,05 bis 0,1 Milligramm Bor pro Liter Wasser gespeist, wodurch insbesondere die Wasserlinse mehr Stärke aufbaut.

Im zweitgenannten Fall werden den aquatischen Pflanzen, insbesondere Wasserlinse etwa 0,5 milligramm Bor pro Liter Wasser zugesetzt, wodurch die Wasserlinse mehr Proteine aufbaut.

Dies ergibt im Ergebnis erst, dass diese Biomasse extrem kurzfristig und daher auch extrem reproduzierbar auf den Synthesegasprozess inhaltsstofflich optmierbar ist.
Und zwar sowohl für den Fall, dass die Biomasse als Eingangsstoff für die Synthesegasfermation, als auch als Eingangsstoff zunächst für die Herstellung von Kohlenstoff durch Heißverkokung und anschließende Synthesegas-Herstellung verwendet wird.

Eine weitere Anwendung besteht darin, dass die Biomasse aus aquatischen Pflanzen gewonnen wird, die in einem geschlossenen Bioreaktor, mit auf einer Vielzahl von Etagen angeordneten Wannen kultiviert wird, und mit Grubenwasser eines Bergwerkes gespeist wird, und dass die geerntete Biomasse dem Synthesegasprozess zugeführt wird, aus welchem in einem Energieumwandlungsprozess elektrische Energie zum Betrieb derjenigen Pumpe oder Pumpen gewonnen wird, die das Grubenwasser aus dem Bergwerk nach oben pumpt.
Mit diesem erfindungsgemäßen Prozess findet die sogenannte Ewigkeitlast (obligatorisches Abpumpen stillgelegter Bergwerke) eine sowohl im ökologischen als auch im ökonomischen Sinne hervorragende Lösung.
Durch die geschlossenen Stoff- und Energiekreisläufe können diese als Ewigkeitslast betriebenen Pumpen plötzlich wirtschaftlich arbeiten.
Eine wesentliche Grundlage der Erfindung ist die Einbeziehung sowohl des belasteten Grubenwassers als auch des im Prozess erzeugten CO₂ als Rohstoffe.
Grubenwässer enthalten in der Regel hohe Eisenwerte.

Genau diese werden insbesondere von der Lemnacea verstoffwechselt und der so ablaufende Fe²⁺ - Fe³⁺ - Prozess ist der wesentliche Grund dafür, dass die Lemnacea als Schwachlichtpflanze überhaupt so viel Biomasse erzeugen kann.

Hier kommt es zu einer erfinderischen Kombination des Stoffbedarfes der Lemnacea als Schwachlichtpflanze und dem Faktum des eigentlich als Wasserbelastung angesehenen Eisengehaltes im Grubenwasser.

In weiterer vorteilhafter Ausgestaltung, insbesondere zur Maximierung der Biomassenerträge für den Einsatz der Synthesegaserzeugung ist angegeben, dass die Synthesegasqualität durch die definierte Zugabe des Elementes Bor oder Bor-Verbindungen beeinflusst werden kann.
Bor-Verbindungen können beispielsweise Borate sein, wie bspw Silicoborate.
Dies ist in Verbindung mit den verwendeten SPEZIELLEN Pflanzen, nämlich aquatischen Pflanzen von erheblichem technologischen Vorteil. Der Grund dafür ist, dass die genannten Pflanzenarten, insbesondere Lemnacea und Spirodela, sowie alle bekannten Wasserlinsen alle 1 bis 5 Tage eine Massenverdopplung vornehmen, und somit große Mengen von Mineralstoffen aufnehmen.

In weiterer vorteilhafter Ausgestaltung ist angegeben, dass das im Prozess anfallendes CO₂ dem Bioreaktor als wuchsfördernder Rohstoff rückgeführt wird. Damit werden die Biomassenerträge bei den genannten aquatischen Pflanzen entscheidend erhöht.

In weiterer vorteilhafter Ausgestaltung ist vorgesehen, dass die bei der Heißverkokung erhaltene Menge an Reinkohlenstoff in das Bergwerk rückverfüllt wird. Damit findet der Prozess eine ganz erhebliche ökologische Wirkung bei dieser Verfahrensausgestaltung, weil damit der Kohlenstoff tatsächlich stofflich gebunden und endgelagert werden kann. Damit stellt dieser fest gebundene Kohlenstoff eine tatsächliche Decarbonisierung im Prozess dar und ist außerdem noch CO₂-Zertifikatfähig.

Für die Gewässerbehandlung ist vorteilhaft ausgestaltet, dass die bei der Heißverkokung erhaltene Menge an Reinkohlenstoff in Aktivkohle umgewandelt wird, und dass die Aktivkohle zur Wasser- oder Abwasser- oder Grubenwasserreinigung verwendet wird.

Eine besonders vorteilhafte Ausgestaltung besteht darin, dass die erzeugte Biomasse einem Prozess zur Biogaserzeugung und/oder Synthesegaserzeugung und/oder Bioenthanolerzeugung so zugeführt wird, dass die Reststoffe sowie die CO₂-haltigen Abgase stofflich aus diesen Prozessen im Kreis gefahren werden, indem diese der Biomassenerzeugungseinrichtung, d.h. den Aquakulturen wieder als Nährstoffe rückgeführt werden. Hierbei findet zwar eine Erweiterung der obigen Anwendung auch auf den Biogasprozess statt, aber dies auch in Bezug auf die nachfolgende weitere Ausgestaltung, indem nämlich zur Abkonzentrierung des Stickstoffes in diesem stofflichen Kreisprozess ein Stickstoff austragender Prozess, wie Synthesegaserzeugung mit Erzeugung von gasförmigem Stickstoff (N₂) zumindest teilweise parallel zum Biogaserzeugungsprozess betrieben wird. In dieser Ausgestaltung wird in dieser Anwendung nämlich der Synthesegasprozess als Stickstoffaustragender Prozess parallel zum Biogasprozess betrieben, weil es bei im Kreis gefahrenen Reststoff ansonsten zu einer Aufkummulation von Stickstoff kommen würde. Wenn man nämlich die Biomasse aus der aquatischen Biomassenerzeugung in den Biogaserzeugungsprozess führt und diese Reststoffe aus dem Biogasprozess wieder als Nährsubstrat immer wieder zurück in die Kulturanlage für die aquatischen Pflanzen zurückführen würde, so würde es mit der Zeit zu einer Aufkumulation von Stickstoff kommen. Dies aber wird durch eine parallele Abzweigung von Biomasse für Synthesegaserzeugung verhindert, indem hierbei Stickstoff gasförmig ausgetragen wird. So können Synthesegaserzeugung und Biogaserzeugung nutzvoll parallel betrieben werden, und von einer Biomassenquelle gespeist werden und die Reststoffe dennoch wieder als Nährstoffe in die Kultureinrichtung zurückgeführt werden.

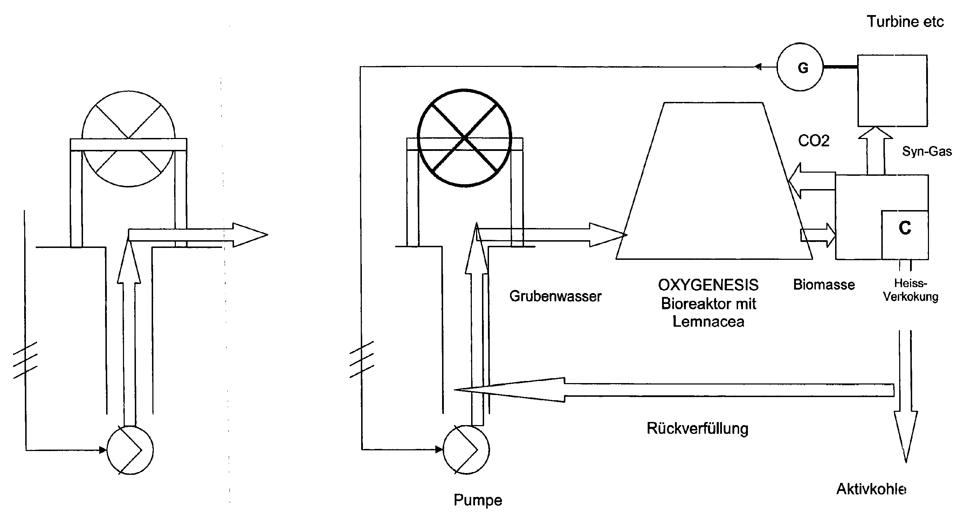

Ein Ausführungsbeispiel zeigt Figur 1.

Hierbei wird eine Anordnung gezeigt, die Grubenwasser aus einem stillgelegten Schacht mittels Pumpe oder Pumpen hochpumpt, damit dies nicht den Grundwasserspiegel so hochdrücken kann, dass die weitläufige Umgebung des Schachtes überflutet wird, wie dies bspw am Niederrhein der Fall wäre. Das hochgepumpte Grubenwasser wird einem Oxygenesis^{®}-Bioreaktor zugeführt, der in einer geschlossenen Außenhülle eine vielfachanordnung von Stellagen zur gestapelten Aufnahme von Wasserwannen aufweist in dem aquatische Pflanzen kultiviert werden.

Die dort erzeugte Biomasse wird nachfolgend einem Synthesegasprozess zugeführt. Dieser erzeugt Synthesegas für einen nachfolgenden Energieerzeugungsprozess. Die erzeugte elektrische Energie wird zum Betrieb der Pumpen verwendet. Beim Prozess entstehendes CO2 wird dem Bioreaktor wiederum als Rohstoff zugeführt.
So entstehen geschlossene Stoff- und Energiekreisläufe.

Der hierbei entstehende Reinkohlenstoff bspw nach Heißverkokung kann als Aktivkohle, bspw auch zur Gewässerreinigung verwendet werden oder als Rohstoff in die alten Bergwerkstollen wieder rückverfüllt werden.

## Patentansprüche

1. Verfahren zur Herstellung von Synthesegas aus Biomasse, bei welchem die Biomasse entweder durch Synthesegas-Fermation oder durch Heissverkokung für den weiteren Prozess vorkonditioniert wird;
**dadurch gekennzeichnet,**
**dass** die Pflanzen, die die Biomasse darstellen, aquatische Pflanzen sind, bei welchen während des Wuchses der aquatischen Pflanzen über die definierte Zugabe des Elementes Bor oder chemische Verbindungen mit Bor zur Nährlösung der Stärkegehalt maximiert wird.

2. Verfahren nach Anspruch 1,
**dadurch gekennzeichnet,**
**dass** im Falle der Heissverkokung die Zufuhr von Bor im Bereich von etwa 0,05 mg bis 0,1 mg pro liter Wasser liegt.

3. Verfahren nach Anspruch 1,
**dadurch gekennzeichnet,**
**dass** im Falle der Synthesegas-Fementation die Zufuhr von Bor im Bereich von etwa 0,1 mg bis 0,5 mg pro liter Wasser liegt.

4. Verfahren nach Anspruch 2 oder 3,
**dadurch gekennzeichnet,**
**dass** die Biomasse im wesentlichen aus Lemnacea, oder Spirodela oder Eichhornia, oder Algen besteht.

5. Verfahren nach einem der Ansprüche 1 bis 4,
**dadurch gekennzeichnet, dass** die Biomasse aus aquatischen Pflanzen gewonnen wird, die in einem geschlossenen Bioreaktor, mit auf einer Vielzahl von Etagen angeordneten Wannen kultiviert wird, und mit Grubenwasser eines Bergwerkes gespeist wird, und dass die geerntete Biomasse dem Synthesegasprozess zugeführt wird, aus welchem in einem Energieumwandlungsprozess elektrische Energie zum Betrieb derjenigen Pumpe oder Pumpen gewonnen wird, die das Grubenwasser aus dem Bergwerk nach oben pumpt.

6. Verfahren nach Anspruch 5,
**dadurch gekennzeichnet,**
**dass** die Pflanzen, die die Biomasse darstellen, aquatische Pflanzen sind, bei welchen während des Wuchses der aquatischen Pflanzen über die definierte Zugabe des Elementes Bor oder chemische Verbindungen mit Bor zur Nährlösung der Stärkegehalt maximiert wird.

7. Verfahren nach einem der vorhergehenden Ansprüchen,
**dadurch gekennzeichnet,**
**dass** im Falle der Heissverkokung die Zufuhr von Bor im Bereich von etwa 0,05 mg bis 0,1 mg pro liter Wasser liegt.

8. Verfahren nach einem der vorhergehenden Ansprüchen,
**dadurch gekennzeichnet,**
**dass** im Falle der Synthesegas-Fementation die Zufuhr von Bor im Bereich von etwa 0,1 mg bis 0,5 mg pro liter wasser liegt.

9. Verfahren nach einem der vorhergehenden Ansprüchen,
**dadurch gekennzeichnet,**
**dass** die Biomasse im wesentlichen aus Lemnacea, oder Spirodela oder Eichhornia, oder Algen besteht.

10. Verfahren nach einem der vorhergehenden Ansprüche,
**dadurch gekennzeichnet,**
**dass** im Prozess anfallendes CO₂ dem Bioreaktor als wuchsfördernder Rohstoff rückgeführt wird.

11. Verfahren nach einem der vorhergehenden Ansprüche,
**dadurch gekennzeichnet,**
**dass** die bei der Heißverkokung erhaltene Menge an Reinkohlenstoff in das Bergwerk rückverfüllt wird.

12. Verfahren nach einem der vorhergehenden Ansprüche,
**dadurch gekennzeichnet,**
**dass** die bei der Heißverkokung erhaltene Menge an Reinkohlenstoff in Aktivkohle umgewandelt wird.

13. Verfahren nach einem der vorhergehenden Ansprüche,
**dadurch gekennzeichnet**
**dass** die Aktivkohle zur Wasser- oder Abwasser- oder Grubenwasserreinigung verwendet wird.

14. Verfahren nach einem der vorhergehenden Ansprüche,
**dadurch gekennzeichnet,**
**dass** die erzeugte Biomasse einem Prozess zur Biogaserzeugung und/oder Synthesegaserzeugung und/oder Bioenthanolerzeugung so zugeführt wird, dass die Reststoffe sowie die CO₂-haltigen Abgase stofflich aus diesen Prozessen im Kreis gefahren werden, indem diese der Biomassenerzeugungseinrichtung, d.h. den Aquakulturen wieder als Nährstoffe rückgeführt werden.

15. Verfahren nach Anspruch 14,
**dadurch gekennzeichnet,**
**dass** zur Abkonzentrierung des Stickstoffes in diesem stofflichen Kreisprozess ein Stickstoff austragender Prozess, wie Synthesegaserzeugung mit Erzeugung von gasförmigem Stickstoff (N₂) zumindest teilweise parallel zum Biogaserzeugungsprozess betrieben wird.
